# EUROPEAN PATENT SPECIFICATION

(11) **EP 3 680 351 B1**
(45) Date of publication and mention of the grant of the patent: **18.09.2024**
(21) Application number: 18850101.9
(22) Date of filing: 29.06.2018
(51) Int. Cl.: C12Q 1/689, G01N 33/569, C12Q 1/6883, G01N 33/68

(54) **PRETERM BIRTH RISK PREDICTION USING CHANGE IN MICROBIAL COMMUNITY IN SAMPLE**
VORHERSAGE DES RISIKOS EINER FRÜHGEBURT DURCH VERÄNDERUNG DER MIKROBENGEMEINSCHAFT IN EINER PROBE
PRÉDICTION DE RISQUE DE NAISSANCE PRÉMATURÉE À L'AIDE D'UN CHANGEMENT DE COMMUNAUTÉ MICROBIENNE DANS UN ÉCHANTILLON

(30) Priority: 04.09.2017 KR 20170112693
(43) Date of publication of application: 15.07.2020
(73) Proprietor: Ewha University-Industry Collaboration Foundation, Seoul 03760 (KR)
(72) Inventor: KIM, Young-Ju, Seoul 07980 (KR); YOU, Young-Ah, Seoul 05085 (KR); DU, Ji-Eun, Yongin-si Gyeonggi-do 16839 (KR); KWON, Eun Jin, Gyeongju-si Gyeongsangbuk-do 38113 (KR); PARK, Mi Hye, Seoul 07985 (KR)
(74) Representative: Germain Maureau
(86) International application number: PCT/KR2018/007435
(87) International publication number: WO 2019/045246

(56) References cited:
- WO-A1-2016/095789
- WO-A1-2019/203422
- KR-B1- 101 831 416
- US-A1- 2015 259 728
- US-A1- 2015 259 728
- US-A1- 2017 114 396
- ARBOLEYA SILVIA ET AL: "Establishment and development of intestinal microbiota in preterm neonates", FEMS MICROBIOLOGY ECOLOGY, vol. 79, no. 3, 15 December 2011 (2011-12-15), NL, pages 763 - 772, XP055799187, ISSN: 0168-6496, DOI: 10.1111/j.1574-6941.2011.01261.x
- J. WALTER ET AL: "Detection of Lactobacillus, Pediococcus, Leuconostoc, and Weissella Species in Human Feces by Using Group-Specific PCR Primers and Denaturing Gradient Gel Electrophoresis", APPLIED AND ENVIRONMENTAL MICROBIOLOGY, vol. 67, no. 6, 1 June 2001 (2001-06-01), pages 2578 - 2585, XP055159976, ISSN: 0099-2240, DOI: 10.1128/AEM.67.6.2578-2585.2001
- NAM HYERAN ET AL: "Analysis of Vaginal Lactic Acid Producing Bacteria in Healthy Women", THE JOURNAL OF MICROBIOLOGY, THE MICROBIOLOGICAL SOCIETY OF KOREA, vol. 45, 1 December 2007 (2007-12-01), XP055799191
- JIN L. ET AL: "Species diversity and relative abundance of vaginal lactic acid bacteria from women in Uganda and Korea", JOURNAL OF APPLIED MICROBIOLOGY, vol. 0, no. 0, 30 August 2006 (2006-08-30), GB, pages - ???, XP055799192, ISSN: 1364-5072, DOI: 10.1111/j.1365-2672.2006.03147.x
- ALINE C. FREITAS ET AL: "The vaginal microbiome of pregnant women is less rich and diverse, with lower prevalence of Mollicutes, compared to non-pregnant women", SCIENTIFIC REPORTS, vol. 7, no. 1, 23 August 2017 (2017-08-23), UK, pages 1 - 16, XP055580189, ISSN: 2045-2322, DOI: 10.1038/s41598-017-07790-9
- FOXMAN BETSY ET AL: "Mycoplasma, bacterial vaginosis-associated bacteria BVAB3, race, and risk of preterm birth in a high-risk cohort", AMERICAN JOURNAL OF OBSTETRICS & GYNECOLOGY, vol. 210, no. 3, 1 March 2014 (2014-03-01), US, pages 226.e1 - 226.e7, XP055799193, ISSN: 0002-9378, DOI: 10.1016/j.ajog.2013.10.003
- FREITAS, A. C. ET AL.: "The Vaginal Microbiome of Pregnant Women Is Less Rich and Diverse, with Lower Prevalence of Mollicutes, Compared to Non-pregnant Women", SCIENTIFIC REPORTS, vol. 7, no. 1, 23 August 2017 (2017-08-23), pages 1 - 16, XP055580189, ISSN: 2045-2322, DOI: 10.1038/s41598-017-07790-9
- HOLST, E. ET AL.: "Bacterial Vaginosis and Vaginal Microorganisms in Idiopathic Premature Labor and Association with Pregnancy Outcome", JOURNAL, OF CLINICAL MICROBIOLOGY, vol. 32, no. 1, January 1994 (1994-01-01), pages 176 - 186, XP055580193, ISSN: 0095-1137
- PETRICEVIC, L. ET AL.: "Characterisation of the Vaginal Lactobacillus Microbiota Associated with Pretem Delivery", SCIENTIFIC REPORTS, vol. 4, 30 May 2014 (2014-05-30), pages 1 - 6, XP055580197, ISSN: 2045-2322, DOI: 10.1038/srep05136

## Description

### [TECHNICAL FIELD]

The present invention relates to a diagnosis method for diagnosing a risk of preterm birth, by detecting change in microbiome from a sample of pregnant woman. More specifically, the present invention relates to a diagnosis method for diagnosing a risk of preterm birth by detecting a Weissella microorganism from a sample of pregnant woman.

### [BACKGROUND ART]

Preterm birth (PTB; delivery before 37 weeks of gestation) generally means delivering a child after 20 weeks and before 37 weeks. Infants born prematurely have a high risk of death, accounting for about 60% of all infant deaths, and even surviving infants require neonatal intensive care due to neurological developmental disorders, respiratory complications, and postnatal growth delays, and the like, and furthermore, have a high morbidity in severe long-term or short-term illness. About 70% of preterm births are caused by preterm labor (PTL) and preterm premature rupture of membranes (PPROM) due to various pathological processes such as intrauterine infection and inflammation. However, mechanisms relating to preterm birth are not yet clearly discovered, and risk factors commonly associated with natural labor are genital infections, multiple pregnancies, 2,3 trimester bleeding and previous preterm birth history, and the like.

The minimum gestational ages to improve the survival rate of premature babies are 27 weeks and the birth weight is 0.9 kg, and the morbidity and death of newborn babies are known to be primarily affected by gestational ages, i.e., maturity, rather than the birth weight. Therefore, when signs of preterm birth occur in the early week, increasing the maturity of newborn babies by delaying the delivery week through appropriate treatment is critical to the health, quality of life and cost of pregnant woman and newborn babies.

To date, for treatment of preterm birth, the labor has been inhibited and the delivery has been delayed, by administering an inhibitor of uterine contraction, antibiotic treatment, and administering steroids and progesterone, to preterm labor pregnant woman. However, it has been known that when preterm labor and preterm premature rupture of membranes occur, the therapeutic effect of antibiotics for intrauterine infection and inflammation is very limited and it is impossible to prevent preterm birth. In addition, cervical cerclage has been performed as treatment and prevention of preterm delivery, because pregnant woman with cervical incompetency has a significant risk of infection and abortion, but this is not a fundamental treatment and is known just to help short-term extension of pregnancy.

Therefore, rather than performing treatment after symptoms of preterm premature rupture of membranes or preterm birth occur, it is necessary to select a preterm birth risk pregnant woman group by predicting a risk of preterm birth in advance, and prevent preterm birth through suitable management for them or develop an effective treatment method. In addition, the earlier the preterm birth is, the more likely it is to leave aftereffects to newborn babies and the degree of aftereffects is severe, and thus it is expected that prediction of preterm birth can significantly reduce incidence of premature infants and their resulting disabilities.

The general approach to predict preterm birth was to identify groups of women who should pay special attention gynecologically, demographically and various syndromes, but this approach has a problem in that it is not sensitive or specific. To overcome this problem, many studies have been conducted to discover a biochemical marker for predicting sudden preterm birth or rupture of membranes, and substances such as plasma estradiol-17 beta, progesterone and C-reactive protein have been nominated as candidates, but this has also been shown to be less accurate. Therefore, it is necessary to develop a marker which is easier to detect and has higher sensitivity and specificity.

Women's vaginal microbiota act as a barrier to bacteria and pathogens, play an important role in women's health, and biochemical and immunological profiles of microbiota may be health indicators of the vaginal environment. Normal microbiota can protect women from genitourinary diseases such as yeast infections, sexually transmitted infections, urinary tract infections and human immunodeficiency virus (HIV) infections. It has been reported that the risk of preterm birth and late abortion increases in women diagnosed with bacterial vaginosis (BV) two times and six times, respectively.

Recent advances in gene sequencing technology have made it possible to investigate human microbiomes at various sites in the human body, but until now, most researches on vaginal microbiota have focused on the composition of vaginal microbiota for healthy women or women with bacterial vaginosis, and there is almost no research on purpose of identifying the relationship between the vaginal microbiota and the risk of preterm birth and utilizing this for diagnosing the risk of preterm birth. Moreover, comparative studies of vaginal microbial characterization in women with early labor pregnant woman and Korean pregnant woman with preterm premature rupture of membranes have not been performed.

Accordingly, the present inventors have characterized vaginal microorganisms in pregnant woman with premature labor or preterm premature rupture of membranes which is a major cause of preterm birth, and have identified vaginal microbial parameters predicting preterm birth using high throughput gene sequencing, thereby completing the present invention.

### [Prior art documents]

(Patent document 1) Korean Patent 10-1559975 (published on October 14, 2015).

It is known from patent applications WO2016/095789 A1 and US2017/114396 A1, a method for determining the risk of adverse pregnancy outcome for a pregnant subject by detecting the elevated level of bacteria from selected bacterial taxa (e.g., genera or species). In particular, WO2016/095789 A1 describes the use of at least three bacterial taxa and US2017/114396 A1 describes the use of at least one bacterial taxon selected from a list included more than 40 taxa, but wherein the bacterial genus *Weissella.*

It is known from document ALINE C. FREITAS ET AL: "The vaginal microbiome of pregnant women is less rich and diverse, with lower prevalence of Mollicutes, compared to non-pregnant women*",* the profiling of the healthy pregnant women microbiome.

It is finally known from document FOXMAN BETSY ET AL: "Mycoplasma, bacterial vaginosis associated bacteria BVAB3, race, and risk of preterm birth in a high risk cohort*",* a study which assess the relationship between preterm birth and selected vaginal bacterial taxa associated with preterm birth either directly or through their association with bacterial vaginosis.

### [DISCLOSURE]

### [TECHNICAL PROBLEM]

The present invention is to contribute to prevention of preterm birth by analyzing vaginal microbiome of pregnant woman and identifying a predictive marker of preterm birth indicating the risk of preterm birth.

For the purpose, an unclaimed embodiment provides a composition for diagnosing a risk of preterm birth of pregnant woman, comprising an agent capable of detecting a Weissella microorganism.

Another unclaimed embodiment provides a kit for diagnosing a risk of preterm birth of pregnant woman, comprising the composition.

A claimed embodiment of the invention provides a method for providing information for diagnosing a risk of preterm birth of pregnant woman, comprising detecting a Weissella microorganism in a sample of pregnant woman.

### [TECHNICAL SOLUTION]

As one aspect, an unclaimed embodiment of the invention relates to a composition for diagnosing a risk of preterm birth of pregnant woman, comprising an agent capable of detecting a Weissella microorganism.

Preferably, the composition for diagnosing may further comprise an agent capable of detecting a microorganism selected from the group consisting of Lactobacillus, Bacteroides and Prevotella.

Preferably, herein, the agent capable of detecting the microorganism may be a primer, probe, antisense oligonucleotide, aptamer or antibody, which is specific for the microorganism.

More preferably, the primer may be a primer capable of amplifying 16S rRNA (16S rDNA) of a microorganism.

As another aspect, an unclaimed embodiment of the invention relates to a kit for diagnosing a risk of preterm birth, comprising the composition.

As other aspect, an unclaimed embodiment of the invention relates to a method for detecting a Weissella microorganism from a sample of pregnant woman, to provide information for diagnosing a risk of preterm birth.

Otherwise, the present invention relates to a method of providing information for diagnosing a risk of preterm birth, comprising detecting a Weissella microorganism from a sample of pregnant woman.

Preferably, the method may further comprise detecting a microorganism selected from the group consisting of Lactobacillus, Bacteroides and Prevotella.

In addition, preferably, the method may comprise
(a) extracting genomic DNA from a sample of pregnant woman,
(b) reacting the extracted genomic DNA with a primer specific for the Weissella microorganism, and
(c) amplifying the reactants.

Furthermore, preferably, the step (c) may be performed through a polymerase reaction.

Moreover, preferably, the step (c) may further comprise comparing the amount of amplified product of the step (c) with the amplified product or cut-off of a sample of normal pregnant woman.

In addition, preferably, the sample may be a sample derived from a cervical vagina of pregnant woman, for example, a vaginal sample, specifically, a vaginal fluid sample.

### [ADVANTAGEOUS EFFECTS]

The composition of an unclaimed embodiment of the invention provides a microbial biomarker for diagnosing a risk of preterm birth, and using it, it is possible to rapidly detect the risk of preterm birth through a simple sampling, and it is possible to develop a rapid diagnosis kit for the risk of preterm birth.

### [BRIEF DESCRIPTION OF THE DRAWINGS]

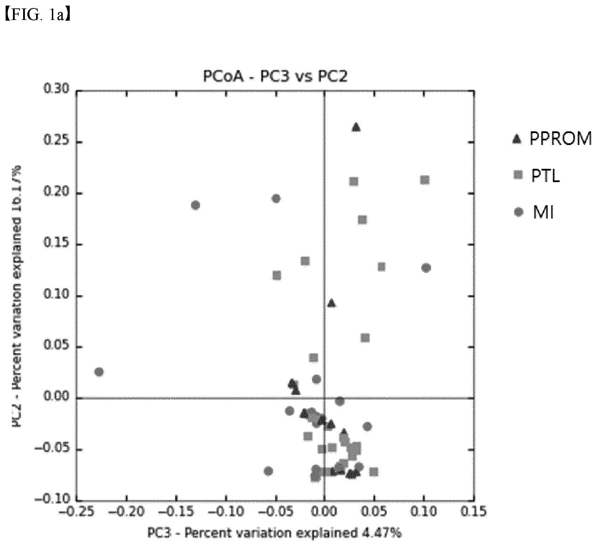

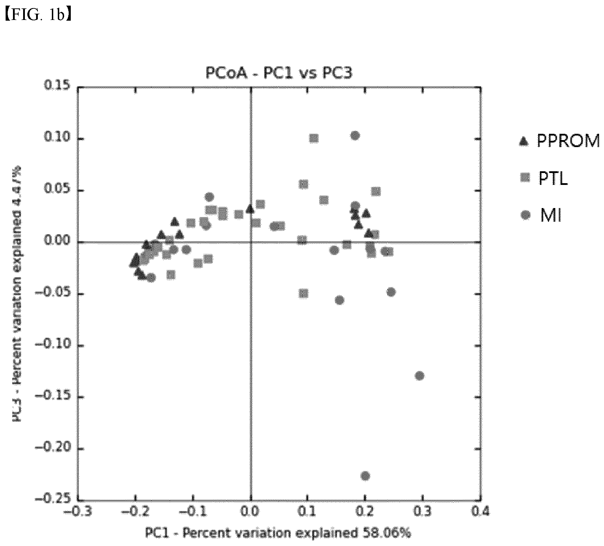

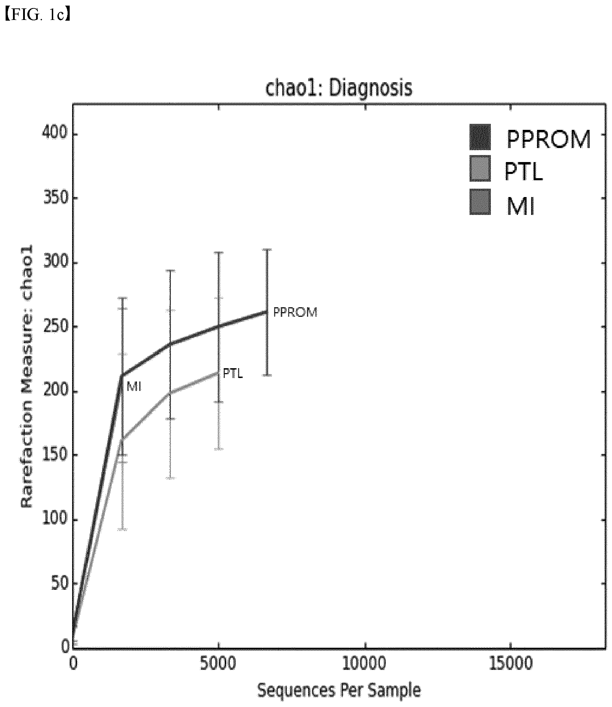

FIG. 1a to FIG. 1c are PCoA graphs obtained by diagnosing pregnant women at first hospitalization. FIG. 1a and FIG. 1b represent principal coordinates analysis (PCoA, n = 65), and FIG. 1c represents Chao 1 for diagnosis (n =65).

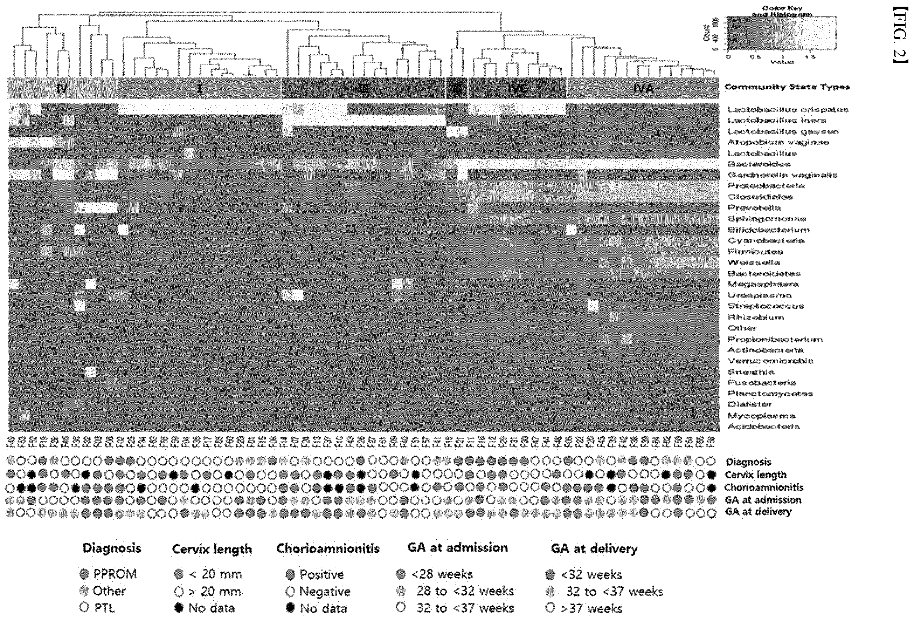

FIG. 2 represents hierarchical clustering for Lactobacillus spp. based microbiome profiles of vaginal samples from 56 pregnant women. CST (Community state types) I, II, III and IV are Lactobacillus-dominated communities, and are composed of L. crispatus, L. gasseri, L. iners or L. jensenii, respectively. Subtype CST IA of CST I was more than 10% of Bacteriodes and more than 10% of L. crispatus. CST IV was described initially as a different species group of non-lactobacilli. Subtype CST IVA of CST IV was more than 10% of Bacteriodes regardless Lactobacillus spp.

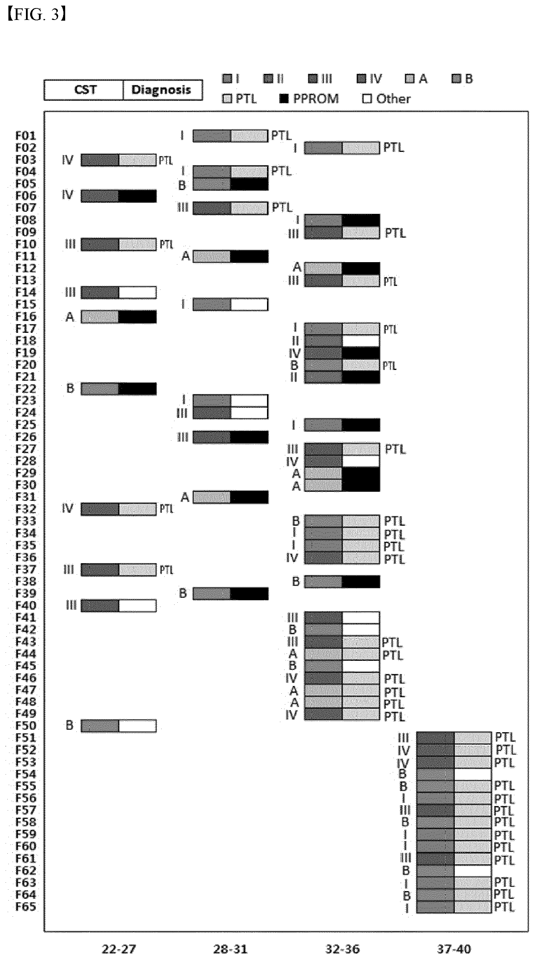

FIG. 3 represents vaginal CST and diagnosis in Korean population. Samples were classified into CSTs on the basis of ward linkage clustering of microbial species data (CST I, orange (I); CST II, red (II); CST III, blue (III); CST IV, purple (IV), and CST IA, sky (A), and IVA, green (B); preterm labor, gray (PTL); preterm premature rupture of membrane, black (PPROM) and other, white (Other)).

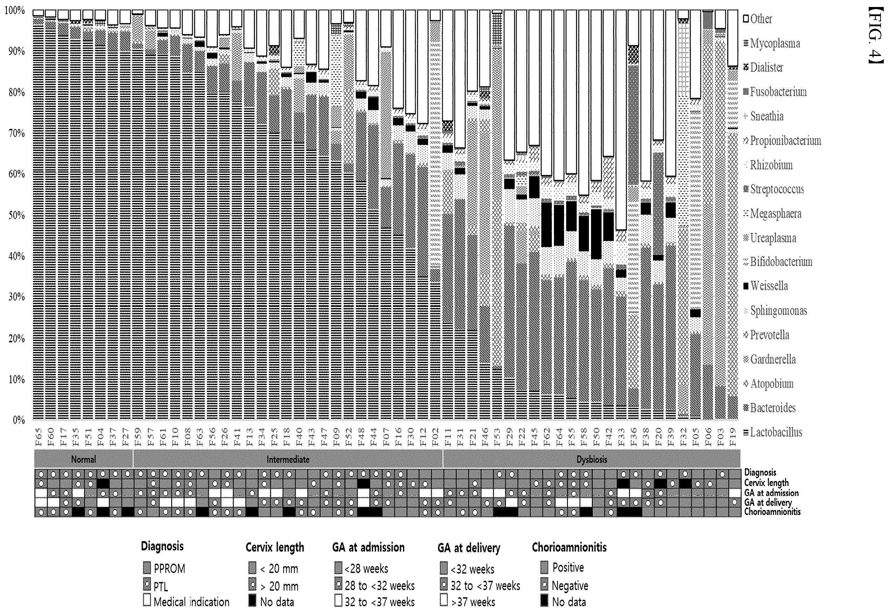

FIG. 4 represents a bar graph of upgma cluster to vaginal bacterial genera of vaginal fluid samples (n = 57) collected from pregnant women PTL (n = 36) or pregnant women PPROM (n =21): normal (>90% of Lactobacillus abundance), intermediate (30 to 90% of Lactobacillus abundance), or dysbiotic (<30% of Lactobacillus abundance).

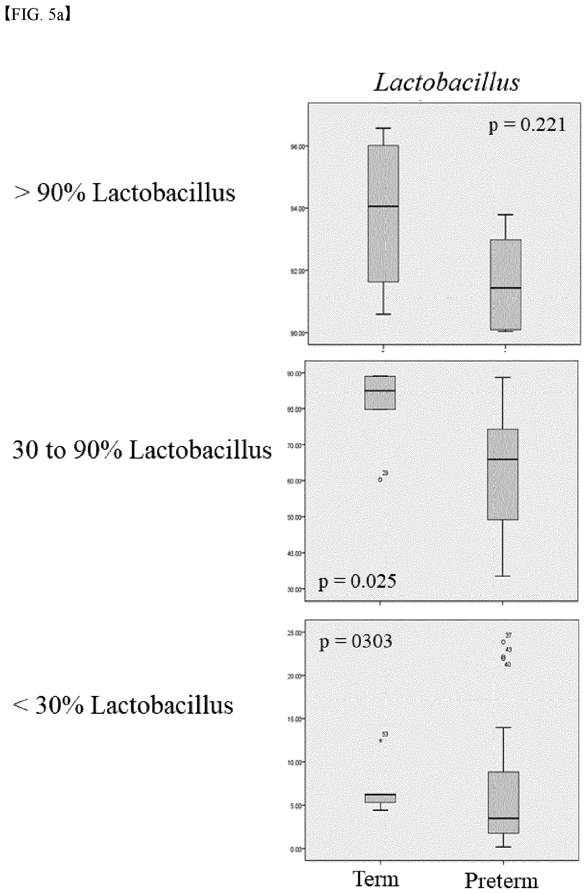

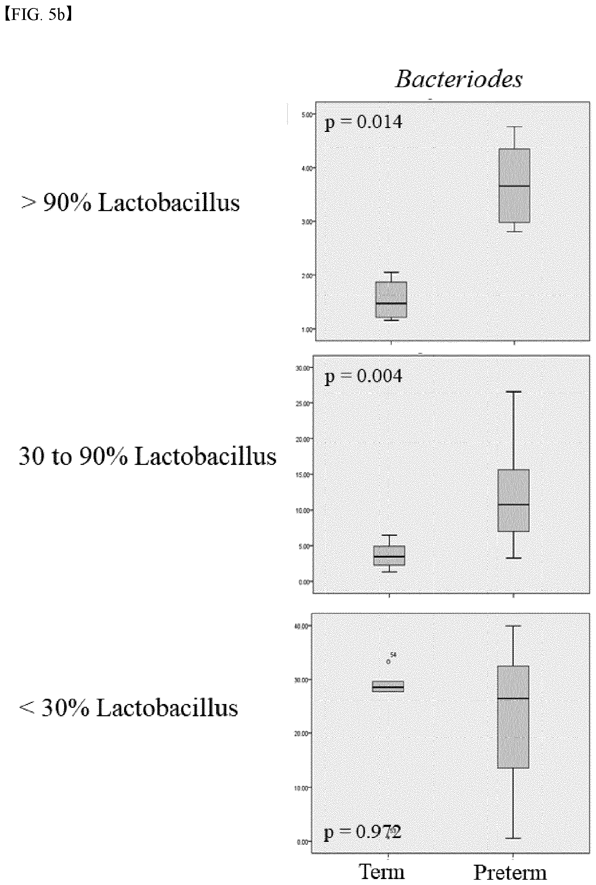

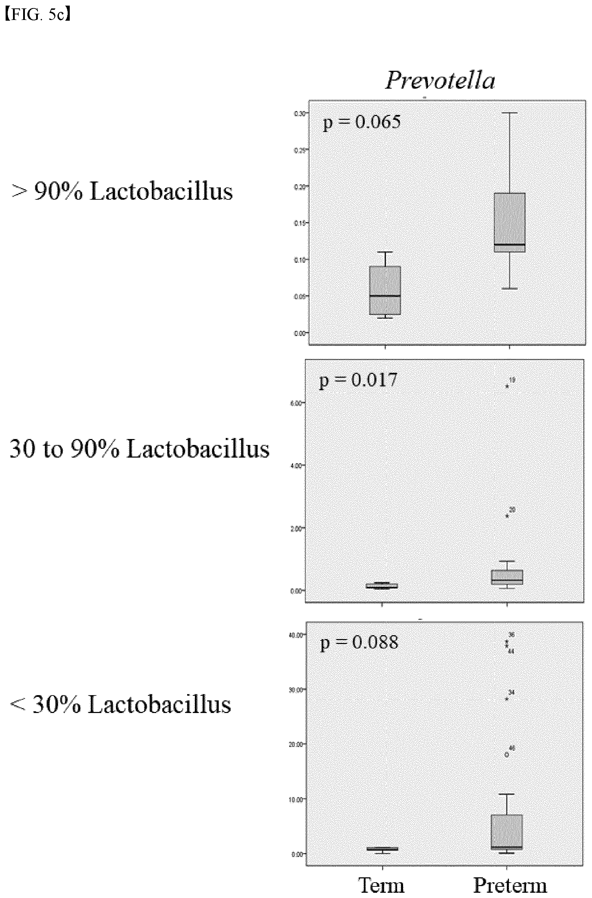

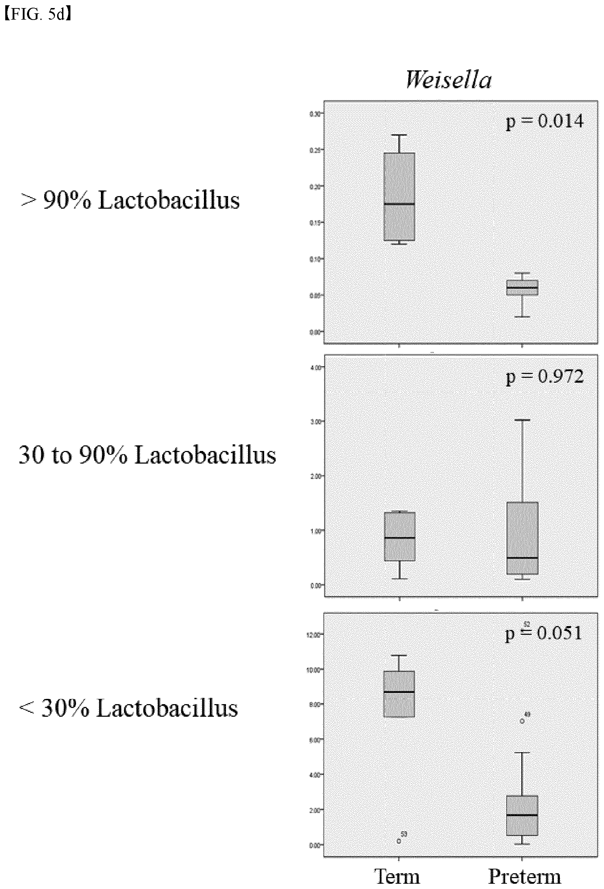

FIG. 5a to FIG. 5d represent the relationship between vaginal microbiome and preterm birth. The result was obtained by comparing the proportional abundance of Lactobacillus (FIG. 5a), Bacteriodes (FIG. 5b), Prevotella (FIG. 5c), and Weissella (FIG. 5d) in normal, intermediate and dysbiotic groups classified by the proportional abundance of Lactobacillus ratio. Mann Whitney U test, p < 0.05.

### [BEST MODE]

Hereinafter, the present invention will be described in more detail.

Herein, as the result of analyzing the effect of the composition of the microbial community on preterm birth in vaginal samples of pregnant women hospitalized due to preterm labor and preterm premature rupture of membranes, it has been confirmed that the reduction of Lactobacillus, the increase of Bacteroides Prevotella and the reduction of Weissella affect the preterm birth. In particular, the reduction of Lactobacillus and the increase of Bacteroides showed the correlation with IL-7 which the present inventors identified as a predictive marker for preterm birth (Korean Patent 10-1559975). Accordingly, the present invention is characterized by providing the distribution change of these microorganisms as a biomarker for predicting preterm birth.

Therefore, herein, a risk of preterm birth of pregnant woman can be diagnosed by detecting a Weissella microorganism in a sample of pregnant woman, and for this, a composition, a kit and a method for detecting a Weissella microorganism are provided. Preferably, the present invention can diagnose a risk of preterm birth of pregnant woman by further detecting Lactobacillus, Bacteroides and/or Prevotella microorganisms, and a composition, a kit (both unclaimed embodiments of the invention) and a method for this are also provided.

Herein, the term, "risk diagnosis" or "risk prediction" refers to determine the whether the possibility of preterm birth occurs less than 37 weeks of gestational weeks, whether the possibility of preterm birth less than 37 weeks is relatively high, or whether signs of preterm birth are possibly shown in less than 37 weeks, for pregnant woman. The present invention may be used for delaying or preventing preterm birth by providing special and appropriate care for pregnant woman with high risk of preterm birth in less than 37 weeks. In addition, the present invention may be clinically used for deciding and selecting the most appropriate treatment method by diagnosing preterm birth of less than 37 weeks in the early stage.

Herein, the term, "marker used for diagnosis, marker for diagnosing or diagnosis marker" or "indicator" means substances that are standard material predicting and classifying pregnant woman with the possibility of the preterm birth (for example, pregnant woman with preterm birth of less than 37 weeks) and pregnant woman with the possibility of normal delivery (for example, pregnant woman delivering at 37 weeks or later), and that are biological organic molecules showing significant differences in samples of pregnant woman predicted as preterm birth than samples of pregnant woman with normal delivery. For the purpose of the present invention, it refers to a Weissella microorganism or community specifically showing the distribution change in samples of pregnant woman predicted as preterm birth, and additionally, refers to a microorganism or community of one or more selected from the group consisting of Lactobacillus, Bacteroides and Prevotella.

Herein, "Weissella" refers to a microorganism composed of species taxonomically belonging to the Weissella genus, or its community. Herein, the Weissella includes not only conventionally reported strains, but also all microorganisms having the sequence homology of preferably 70% or more, more preferably 80% or more, more preferably, 90% or more, or most preferably 95% or more, when comparing 16S rRNA sequence with conventionally reported Weissella fall in the scope of the present invention.

Herein, "Lactobacillus" refers to a microorganism composed of species belonging to the Lactobacillus genus taxonomically or its community. Herein, the Lactobacillus includes not only conventionally reported strains, but also all microorganisms having the sequence homology of preferably 70% or more, more preferably 80% or more, more preferably, 90% or more, or most preferably 95% or more, when comparing 16S rRNA sequence with conventionally reported Lactobacillus fall in the scope of the present invention.

Herein, "Bacteroides" refers to a microorganism composed of species belonging to the Bacteroides genus taxonomically or its community. Herein, the Bacteroides includes not only conventionally reported strains, but also all microorganisms having the sequence homology of preferably 70% or more, more preferably 80% or more, more preferably, 90% or more, or most preferably 95% or more, when comparing 16S rRNA sequence with conventionally reported Bacteroides fall in the scope of the present invention.

Herein, "Prevotella" refers to a microorganism composed of species belonging to the Prevotella genus taxonomically or its community. Herein, the Prevotella includes not only conventionally reported strains, but also all microorganisms having the sequence homology of preferably 70% or more, more preferably 80% or more, more preferably, 90% or more, or most preferably 95% or more, when comparing 16S rRNA sequence with conventionally reported Prevotella fall in the scope of the present invention.

Herein, the term, "agent capable of detecting" means substances which can be used for detecting the presence of Weissella, Lactobacillus, Bacteroides and/or Prevotella as diagnosis markers for a risk of preterm birth in a sample. For example, it may be a primer, probe, antisense oligonucleotide, aptamer or antibody or the like, capable of specifically detecting organic biomolecules such as protein, nucleic acid, lipid, glycolipid, glycoprotein or saccharide (monosaccharide, disaccharide, oligosaccharide, and the like) which are specifically present in Weissella, Lactobacillus, Bacteroides and/or Prevotella.

Preferably, herein, the agent, of the unclaimed composition, capable of detecting may be a primer capable of detecting Weissella, Lactobacillus, Bacteroides and/or Prevotella. It is preferable that the primer specifically detects the genomic sequence of Weissella, Lactobacillus, Bacteroides and/or Prevotella and does not bind to the genomic sequence of other microorganisms specifically. More preferably, it may be a primer capable of amplifying 16S rRNA of one or more of microorganisms selected from the group consisting of Weissella, Lactobacillus, Bacteroides and Prevotella.

Herein, the term, "primer" means 7 to 50 nucleic acid sequences which can form a base pair complementary to a template strand and function as a starting point for template strand copying. The primer is commonly synthesized, but it may be used from naturally produced nucleic acids. The sequence of the primer is not necessarily accurately same as the template sequence, and it is enough to be sufficiently complementary and capable of being hybridized with the template. Additional characteristics which do not change basic properties of the primer may be mixed. The example of additional characteristics which can be mixed includes methylation, capping, substitution of one or more nucleic acids to homologues and modifications between nucleic acids, and the like, but not limited thereto. Preferably, the primer of an unclaimed composition of the invention may be a primer capable of amplifying 16S rRNA of Weissella, Lactobacillus, Bacteroides and/or Prevotella microorganisms.

Herein, the term, "16S rRNA" is rRNA consisting of 30S subunits of prokaryotic ribosome, and can identify microorganisms through sequencing, since there are a conserved region which is common to all species and a hypervariable region which can classify a specific species. In particular, since there is little diversity among homogeneous species, but there is diversity among other species, prokaryotes can be usefully identified by comparing sequences of 16S rRNA. In addition, 16S rDNA is a gene encoding 16S rRNA, and therefore microorganisms can be identified by utilizing 16S rDNA.

As one preferable unclaimed embodiment, herein, the primer, of the unclaimed composition, may be used for amplifying a 16S rRNA (or 16S rDNA) sequence specific to Weissella, Lactobacillus, Bacteroides and/or Prevotella, and through the production of desired products as the result of sequence amplification, the presence of Weissella, Lactobacillus, Bacteroides and/or Prevotella may be detected. As the sequence amplification method using a primer, various methods known in the art may be used. For example, polymerase chain reaction (PCR), reverse transcriptase-polymerase chain reaction (RT-PCR), multiplex PCR, touchdown PCR, hot start PCR, nested PCR, booster PCR, real-time PCR, differential display PCR (DD-PCR), rapid amplification of cDNA ends (RACE), inverse polymerase chain reaction, vectorette PCR, TAIL-PCR (thermal asymmetric interlaced PCR), ligase chain reaction, repair chain reaction, transcription-mediated amplification, self-sustained sequence replication, or selective amplification reaction of a target sequence may be used.

In addition, herein, the agent, of the unclaimed composition, for detecting a microorganism may be an antibody, and the corresponding microorganism may be detected using an immunological method based on an antigen-antibody reaction. The analysis methods for this include western blotting, ELISA (enzyme linked immunosorbent assay), radioimmunoassay (RIA), radioimmunodiffusion, Ouchterlony immunodiffusion, rocket immunoelectrophoresis, immunohistostaining, immunoprecipitation assay, Complement Fixation Assay, FACS (Fluorescence activated cell sorter), or a protein chip, or the like, but not limited thereto.

In addition, molecules and immunological methods widely used in the art may be used for detecting a microorganism of the present invention.

The composition for diagnosing comprising the agent for detecting a microorganism of the unclaimed embodiment of the invention may be provided as implemented in the form of a diagnosis kit. The kit according to the unclaimed embodiment of the invention comprises a detecting agent such as a primer, probe, antisense oligonucleotide, aptamer or antibody or the like for detecting the corresponding microorganism, and may also comprise one or more kinds of other component compositions, solutions or devices which are suitable for the analysis method.

As one specific example, herein, the kit of the unclaimed embodiment of the invention comprising a primer specific to Weissella, Lactobacillus, Bacteroides and/or Prevotella may be a kit comprising essential components for performing an amplification reaction such as PCR and the like. For example, the kit for PCR may comprise a test tube or other appropriate container, reaction buffer, deoxynucleotides (dNTPs), enzyme such as Taq-polymerase and reverse transcriptase, DNase, RNAse inhibitor, DEPC-water or sterile water, or the like.

In addition, the present invention provides a method for diagnosing a risk of preterm birth of pregnant woman, by detecting a Weissella microorganism from a sample of pregnant woman.

In other words, the present invention provides a method for detecting a Weissella microorganism from a sample of pregnant woman, to provide information for diagnosing a risk of preterm birth of pregnant woman. Otherwise, the present invention provides a method for providing information for diagnosing a risk of preterm birth, comprising detecting a Weissella microorganism from a sample of pregnant woman.

Preferably, the method may further comprise a step of detecting Lactobacillus, Bacteroides, and Prevotella microorganisms.

As one preferable example, the method may be implemented by comprising the following steps.
(a) extracting genomic DNA from a sample of pregnant woman,
(b) reacting the extracted genomic DNA with a primer specific for the Weissella microorganism, and
(c) amplifying the reactants.

In the step (a), "sample of pregnant woman" is collected from a women's body to diagnose a risk of preterm birth, and preferably, it may be a cell sample, tissue sample or body fluid sample, which is collected from the vagina of pregnant woman, and for example, it may be a vaginal sample, or vaginal fluid sample.

In the step (b), the extraction of the genomic DNA from the sample of pregnant woman may be performed by applying common technology known in the art, and the primer specific to Weissella, Lactobacillus, Bacteroides and/or Prevotella microorganisms is as described above.

In the step (c), as the method for amplifying the reactants, common amplification technologies known in the art, for example, polymerase chain reaction, reverse transcriptase-polymerase chain reaction, multiplex PCR, touchdown PCR, hot start PCR, nested PCR, booster PCR, real-time PCR, differential display PCR, rapid amplification of cDNA ends, inverse polymerase chain reaction, vectorette PCR, TAIL-PCR, ligase chain reaction, repair chain reaction, transcription-mediated amplification, self-sustained sequence replication, or selective amplification reaction of a target sequence may be used, but the scope of the present invention is not limited thereto.

In the step (c), comparing the amount of the amplification product of reactants with the amplification product or cut-off of the normal pregnant woman sample may be further performed, and it may be determined at high risk of preterm birth, when the amplified product of Weissella is significantly reduced in the sample of pregnant woman compared to the amplified product or cut-off of the sample of normal pregnant woman.

Furthermore, the method may further comprise detecting Lactobacillus, Bacteroides and Prevotella microorganisms, and when the amplification product of Lactobacillus is significantly reduced, or the amplification product of Bacteroides or Prevotella is significantly increased, compared to the amplification product or cut-off of the normal pregnant woman sample, the corresponding pregnant woman may be determined at high risk of preterm birth.

### [MODE FOR INVENTION]

Hereinafter, the present invention will be described in more detail by the following examples. However, these examples are intended to exemplarily illustrate one or more specific embodiments.

### Example 1. Materials and methods

The present study was performed after being approved for all procedures and processes in the experiment from Institutional Review Board of the Ewha Womens University Mokdong Hospital's (certificate no. ECT 06-127-7), Samsung Medical Center (SMC 2014-06-094-003), Konkuk University Medical Center (KUH1040034), Seoul St. Mary's Hospital (KC14TIMI0591), and SMG-SNU Boramae Medical Center (16-2014-94), and performed the experiment according to the guidelines of the Institutional Review Board. All participants provided written informed consent.

### Example 2. Study design

Vaginal fluid samples were collected from 65 singleton pregnant woman who were in the preterm labor (PTL) and/or preterm premature rupture of membranes (PPROM) between 22+0 and 36+6 weeks of gestation at first hospitalization from five Seoul sites (Ewha Womens University Mokdong Hospital, Samsung Medical Center, Konkuk University Hospital, Yeouido St. Mary's Hospital, and SMG-SNU Boramae Medical Center). Preterm labor was diagnosed as the presence of uterine contractions of sufficient frequency and intensity to effect progressive effacement and dilatation of the cervix, occurring at 20-37 weeks' gestation. Preterm premature rupture of membranes was confirmed by evidence of vaginal fluid pooling, a positive nitrazine test (elevated pH) and fern test. Vaginal fluid samples were collected from the posterior fornix using swabs and divided into two tubes for ELISA and 16S rRNA gene sequencing. Samples collected were placed to -80°C storage within 30 minutes. Women with multiple births, major birth defects, and pregnancy complications were excluded. Gestational age was determined using the first day of the last menstrual period as well as ultrasound examination. The cervical length is defined as the length between internal cervical os and the external cervical os. After delivery, histological chorioamnionitis was diagnosed on placental pathology. Demographic and clinical characteristics were collected from patient charts, and neonatal outcome data collected included Apgar score, birth weight, gender, admission to neonatal intensive care unit.

### Example 3. Fetal fibronectin and cytokine analysis

The vaginal fluid samples after thawing on ice were centrifuged at 3000xg for 2 min, and the supernatant was collected into a new microcentrifuge tube to remove any cellular debris. The human fetal fibronectin concentration was in cell-free supernatants using Human fetal fibronectin kit (Cusabio, Wuhan, Hubei, China). The absorbance was read using Model 680 Microplate reader (Bio-rad Lab. Inc., Hercules, CA, USA) at 450 nm. The level of cytokines in cell-free supernatants was measured by Human Magnetic Luminex Screening Assay (Luminex Co., Austin, Texas, USA) and Bio-Plex 200 system (Bio-Rad Lab. Inc.). Analyte-specific Luminex Screening Assays were performed for 13 analytes of Interleukin (IL) -1β, IL-4, IL-5, IL-6, IL-7, IL-8, IL-10, IL-13, IL-17α, G-CSF, TNF-α, MIP-1α and MIP-1β. Samples were analyzed on 96-well plates at two dilutions (1:1 and 1:50) optimized for detection of analytes within the range of the standards as specified by Luminex human premixed analyte kit.

### Example 4. 16S rRNA sequencing and taxonomy assignment

Bacterial DNA was extracted from samples using a PowerMax Soil DNA Isolation Kit (MOBIO, Carlsbad, CA, USA) followed the manufacturer's protocol. In bacterial genomic DNA, V3-V4 hypervariable regions (519F-816R) were amplified according to the Illumina 16S metagenomic sequencing library protocols (Illumina, San Diego, CA, USA). The barcoded fusion primer sequences used for amplifications were 519F (5 'CCTACGGGNGGCWGCAG 3') and 806R (5 'GACTACHVGGTATCTAATCC 3'). The final purified product is then quantified using qPCR according to the qPCR Quantification Protocol Guide (KAPA Library Quantificatoin kits for Illumina Sequencing platforms) and qualified using the LabChip GX HT DNA High Sensitivity Kit (PerkinElmer, Massachusetts, USA). The paired-end (2×300 bp) sequencing was performed by the Macrogen using the MiSeq^{™} platform (Illumina). OUT (Open-Reference Operation Taxonomic Unit) picking was performed at the 97% sequence similarity level and OUT sequence were assigned taxonomy using type - strain database in StrainInfo. Sequence data were analyzed alpha and beta diversity using the QIIIME software package (v. 1.8).

### Example 5. Statistical analysis

Clinical characteristics and the outcomes of pregnant women between PTL, PPROM, and medical indication group were analyzed using the Fisher's exact test for categorical variables, and Kruskal-Wallis test for continuous variables. Mann-Whitney U test was conducted to compare differences of vaginal microbial proportional abundance by QIIME. The effects of Lactobacillus abundance on bacterial genera, the number of species, and a diversity were assessed using Kruskal-Wallis test and Spearman correlation. Statistical analyses were conducted using the SPSS software ver. 21.0 (IBM, Armonk, NY, USA). All of the analyses were two-tailed, and a p < 0.05 was considered to indicate statistical significance.

### Results

### 1. General characteristics of study subjects

Table 1 shows the clinical characteristics of 65 singleton pregnancies. They were admitted with PTL (n = 36), PPROM (n = 21), or MI (n = 8) between 22+0 and 36+6 weeks of gestation. There were preeclampsia (n =3), incompetent internal Os of cervix (n = 2), short cervix (n = 2), and HELLP syndrome (n = 1) in MI group. In PTL, PPROM, and MI groups, the means of maternal age were 31.7, 33.6, and 30.6 years old, respectively. Gestational ages at admission between three groups were not significantly different, while gestational ages at delivery, interval from admission to delivery and baby weight were significantly different (p < 0.05). The prevalence rate of chorioamnionitis was no significantly different between three groups.

**[Table 1]**

| Clinical characteristics of study subjects | | | | | | | | | | | |
|---|---|---|---|---|---|---|---|---|---|---|---|
| | | | Total (n=65) | | PTL (n=36) | | PPROM (n=21) | | MI (n=8) | | P value |
| | | | Mean | SD | Median | IQR | Median | IQR | Median | IQR | |
| Gestational age at admission | | | 30.2 | 4.2 | 32.2 | 8.2 | 30.3 | 7.3 | 27.3 | 3.6 | 0.27 |
| | Maternal age | | 32.1 | 4.6 | 33.0 | 7.0 | 33.0 | 3.5 | 30.0 | 5.0 | 0.18 |
| | Fetal fibronectin | | 78.6 | 125.8 | 26.8 | 52.3 | 42.4 | 36.5 | 33.1 | 58.3 | 0.67 |
| | Cervix length (mm) | | 21.8 | 14.8 | 17.7 | 25.0 | 22.3 | 19.3 | 26.5 | 35.5 | 0.34 |
| Gestational age at delivery | | | 33.2 | 4.5 | 35.5 | 5.1 | 32.2 | 6.6 | 31.6 | 5.3 | < 0.01 |
| Interval from admission to delivery | | | | | | | | | | | 0.03* |
| | | < 7days (n) | 36 | | 18 | (27.7) | 15 | (23.1) | 3 | (4.6) | |
| | | 7 days to 28 (n) days | 9 | | 3 | (4.6) | 5 | (7.7) | 1 | (1.6) | |
| | | > 28 days (n) | 20 | | 15 | (23.1) | 1 | (1.5) | 4 | (6.2) | |
| | Chorioamnionitis | | | | | | | | | | 0.16* |
| | | Positive | 23 | | 9 | (17.3) | 12 | (23.1) | 2 | (3.9) | |
| | | Negative | 29 | | 15 | (28.9) | 8 | (15.4) | 6 | (11.5) | |
| | Birth weight | | 2082.1 | 865.4 | 2525.0 | 1100.0 | 1640.00 | 1345.0 | 1800.0 | 1677.5 | < 0.01 |
| | Apgar score, 1 min | | 7.3 | 2.6 | 8.0 | 2.5 | 7.00 | 4.0 | 8.5 | 2.5 | 0.04 |
| | Apgar score, 5 min | | 8.5 | 2.0 | 9.0 | 1.5 | 9.00 | 2.5 | 9.5 | 1.0 | 0.26 |
| | Sex | | | | | | | | | | 0.13* |
| | | Male | 34 | | 15 | (23.1) | 13 | (20.0) | 6 | (9.2) | |
| | | Female | 31 | | 21 | (32.3) | 8 | (12.3) | 2 | (3.1) | |

In the Table 1, total data were shown as mean ± SD and other data were shown as median and interquartile range (IQR), and as n, (%) for categorical data. Significant differences of data were analyzed by Kruskal-Wallis test and nominal scales were analyzed by χ2 test (p < 0.05). PTL represents preterm labor, and PPROM represents preterm premature rupture of membranes.

### 2. Diversity of the vaginal microbial community in the pregnant women with risk of preterm birth

To characterize the vaginal microbial composition at admission in the pregnant women with risk of preterm birth, vaginal samples from 57 subjects except samples with medical indication were analyzed. The data set of the study was consisted of 1,055,829 gene sequences from the vaginal fluid samples, with an average of 16,243 reads per sample (range, 3,216 to 28,407).

The sequence data of vaginal samples were subjected to principle coordinates analysis and measurement of Chao 1 according to diagnosis (PTL, PPROM, and Medical indication) (FIG. 1a to FIG. 1c). To investigate the bacterial diversity between PTL and PPROM samples, the mean of Shannon index and Simpson index were calculated. The vaginal samples of women with PPROM presented a significantly higher Shannon index (richness within the microbial community), and Simpson index (microbial diversity) (p < 0.05, Table 2). Lactobacillus was more abundant in women with PTL than PPROM, while, Bacteriodes, Prevotella, Sphingomonas, Weissella, Streptococcus, Rhizobium, Ureaplasma, Propionibacterium and Fusobacterium were more abundant in women with PPROM than PTL (p < 0.05). However, the amount of Atopobium, Gardnella, Mycoplasma, and Sneathia was not different between samples of PTL and PPROM.

**[Table 2]**

| Comparison of vaginal microbial community according to diagnosis of pregnant women at first admission | | | | | | | | |
|---|---|---|---|---|---|---|---|---|
| | | Total (=57) | | PTL (n =36) | | PPROM (n = 21) | | P value |
| | | Means | SD | Median | IQR | Median | IQR | |
| Alpha diversity | | | | | | | | |
| | Shannon index | 3.136 | 1.726 | 2.171 | 2.031 | 4.324 | 3.113 | 0.008 |
| | Simpson index | 0.630 | 0.267 | 0.573 | 0.458 | 0.865 | 0.346 | 0.010 |

| Taxonomy | | | | | | | | |
|---|---|---|---|---|---|---|---|---|
| | Lactobacillus | 45.526 | 36.370 | 67.130 | 81.995 | 21.940 | 40.390 | 0.004 |
| | Atopobium | 2.458 | 10.189 | 0.010 | 0.040 | 0.000 | 0.040 | 0.772 |
| | Gardnerella | 4.488 | 11.384 | 0.040 | 2.290 | 0.020 | 0.550 | 0.784 |
| | Bacteroides | 14.702 | 12.146 | 5.005 | 11.480 | 23.190 | 24.315 | < 0.001 |
| | Prevotella | 2.967 | 8.179 | 0.260 | 0.543 | 0.840 | 1.530 | 0.011 |
| | Sphingomonas | 2.626 | 2.388 | 0.930 | 2.330 | 4.180 | 4.805 | 0.002 |
| | Bifidobacterium | 2.491 | 10.172 | 0.000 | 0.020 | 0.000 | 0.065 | 0.842 |
| | Sneathia | 0.333 | 2.359 | 0.000 | 0.000 | 0.000 | 0.010 | 0.106 |
| | Weissella | 1.830 | 2.856 | 0.315 | 1.220 | 1.530 | 2.400 | 0.030 |
| | Megasphaera | 1.032 | 4.792 | 0.000 | 0.010 | 0.000 | 0.020 | 0.495 |
| | Ureaplasma | 1.075 | 4.192 | 0.010 | 0.148 | 0.200 | 1.805 | 0.014 |
| | Rhizobium | 1.108 | 1.182 | 0.345 | 0.890 | 1.390 | 1.985 | 0.009 |
| | Streptococcus | 1.261 | 4.979 | 0.120 | 0.295 | 0.440 | 0.575 | 0.010 |
| | Propionibacterium | 0.835 | 1.450 | 0.235 | 0.753 | 0.880 | 1.090 | 0.029 |
| | Mycoplasma | 0.236 | 1.085 | 0.000 | 0.008 | 0.000 | 0.000 | 0.014 |
| | Dialister | 0.268 | 0.746 | 0.000 | 0.048 | 0.010 | 0.160 | 0.299 |
| | Fusobacterium | 0.242 | 0.580 | 0.060 | 0.195 | 0.240 | 0.325 | 0.008 |

In the Table 2, total data were shown as mean ± SD and other data were shown as median and interquartile range (IQR). Significant differences of data were analyzed by Mann-Whitney U test (p < 0.05).

### 3. Vaginal microbial profiles in pregnant women with risk of preterm birth

The vaginal microbial profiles were clustered into L. crispatus (n = 12), L. gasseri (n = 2), and L. iners (n = 13), corresponding to CST (community state types) I, II, and III, respectively, according to Lactobacillus spp. (FIG. 2). Subgroup IVA of CST IV was consisted primarily of Bifidobacterium, Dialister, Streptococcus, and Bacteroides (n = 21). The subtype IA of CST I was dominated by Bacteriodes with L. crispatus (n = 9). Interestingly, pregnant women with CST I microorganisms did not delivered below 28 gestational week, and five women with mean 9.4% of Weissella in IVA were delivered at term, and there was no term delivered woman in CST IA (FIG. 3).

When the dominance of Lactobacillus was shown as the percentage to the total bacteria, samples were classified into normal (> 90% Lactobacillus, n = 10), intermediate (30 to 90% Lactobacillus, n = 23), or dysbiotic (< 30% Lactobacillus, n = 24). Figure 4 shows the abundance of main microbiota at admission. 10 Women only diagnosed as PTL showed the normal microbial community (Table 3). Intermediate and dysbiotic microbial community embodied samples of women diagnosed as PTL or PPROM. The more Lactobacillus abundance decreased, the more Bacteriodes, Gardnerella, Prevotella, Sphigomonas, Weissella, Streptococcus, Rhizobium, Propionibacterium, Sneathia, and Fusobacterium significantly increased (p < 0.01) (Fig. 4). Interestingly, prevalence of chorioamnionitis was higher in dysbiotic microbial community of vaginal than in normal and intermediate (Table 3).

**[Table 3]**

| Clinical characteristics of study subjects according to classification by Lactobacillus portion | | | | | | | | | | | |
|---|---|---|---|---|---|---|---|---|---|---|---|
| | | | All (n = 57) | | > 90% (n = 10) | | 30 to 60% (n = 23) | | <30% (n = 24) | | P Value |
| | | | Mean | SD | Median | IQR | Median | IQR | Median | IQR | |
| GA at admission | | | 30.5 | 4.2 | 32.0 | 4.4 | 32.7 | 8.8 | 29.6 | 6.0 | 0.840 |
| | Maternal age | | 32.4 | 4.8 | 34.0 | 4.0 | 33.0 | 6.0 | 33.0 | 7.5 | 0.207 |
| | Fetal fibronectin | | 81.4 | 129.7 | 41.3 | 155.5 | 40.2 | 58.8 | 32.5 | 39.6 | 0.665 |
| | Cervix length | | 21.4 | 14.2 | 16.6 | 22.7 | 22.3 | 17.0 | 20.0 | 27.6 | 0.668 |
| Diagnosis | | | | | | | | | | | 0.014 |
| | | PTL | 36 | (63.2) | 9 | (15.8) | 16 | (28.1) | 11 | (19.3) | |
| | | PPROM | 21 | (36.8) | 0 | (0.0) | 8 | (14.0) | 13 | (22.8) | |
| GA at delivery | | | 33.3 | (4.7) | 35.2 | 1.9 | 34.5 | 3.1 | 33.8 | 7.7 | 0.642 |
| | Interval | | | | | | | | | | 0.266 |
| | | < 7days (n) | 33 | (57.9) | 4 | (7.0) | 14.0 | (24.6) | 15 | (26.3) | |
| | | 7 days to 28 days (n) | 8 | (14.0) | 2 | (3.5) | 1.0 | (1.8) | 5 | (8.8) | |
| | | > 28 days (n) | 16 | (28.1) | 3 | (5.3) | 9.0 | (15.8) | 4 | (7.0) | |
| Chorioamnioniti s | | | | | | | | | | | 0.039 |
| | | Positive | 21 | (47.7) | 1 | (2.3) | 7 | (15.9) | 13 | (29.5) | |
| | | Negative | 23 | (52.3) | 5 | (11.4) | 12 | (27.3) | 6 | (13.6) | 0.115 |
| | Birth weight | | 2097.3 | 875.9 | 3020.0 | 1400.0 | 2315.0 | 1040.0 | 1880.0 | 1635.0 | 0.264 |
| | Apgar score, 1 min | | 7.2 | 2.6 | 9.0 | 8.0 | 1.0 | 5.5 | 7.0 | 1.8 | 0.462 |
| | Apgar score, 5 min | | 8.4 | 2.1 | 9.0 | 9.0 | 1.0 | 2.0 | 9.0 | 2.3 | 0.867 |
| | Sex | | | | | | | | | | 0.954 |
| | | Male | 28 | (49.1) | 4 | (7.0) | 12 | (21.1) | 12 | (21.1) | |
| | | Female | 29 | (50.9) | 5 | (8.8) | 12 | (21.1) | 12 | (21.1) | |

In the Table 3, three groups were separated by Lactobacillus proportion. Total data were shown as mean ± SD and other data were shown as median and interquartile range (IQR), and as n, (%) for categorical data. Significant differences of data were analyzed by Kruskal-Wallis test and nominal scales were analyzed by χ2 test (p < 0.05).

### 4. Association between vaginal microorganisms and cytokine concentration

The association between vaginal bacterial communities at admission and gestational ages was analyzed. In normal Lactobacillus community, Bacteriodes and Prevotella were more abundant, while Weissella was more depletion in the vaginal fluid of preterm delivered women than in term delivered women (FIG. 5a to FIG. 5d). In intermediate Lactobacillus community, Bacteriodes and Prevotella were more abundant, while Lactobacillus was more depletion in the vaginal fluid of preterm delivered women (p < 0.05). In the dysbiotic Lactobacillus group, Prevotella was substantially abundant, while Weissella was more depletion in the vaginal fluid of preterm delivered women (p < 0.05). In addition, high proportion of Prevotella (r² = 0.331, p < 0.05) and low proportion of Lactobacillus (r² = -0.503, p < 0.05) were associated with prevalence of chorioamnionitis.

To search microbial indicator related to preterm birth, the concentrations of 13 kinds of inflammatory cytokines and the correlation between the dominance of Lactobacillus, Bacteriodes, Prevotella and Weissella were assessed. The IL-1β level (n = 55, mean 816.7 ± 1483.4 pg/ml) had marginally negative correlation with Weissella proportion (r2 = -0.388, p < 0.05). The level of IL-7 (n = 55, mean 7.7 ± 5.6 pg / ml) showed the negative correlation with the dominance of Lactobacillus (r² = -0.404, p <0.01) and the gestational ages (r2 = -0.318, p <0.05), and showed the positive correlation with the dominance of Bacteroides (r2 = 0.307, p <0.05). There are no correlation vaginal microbiome and other cytokines.

### 5. Conclusion

Herein, the vaginal microbiomes were compared for Korean women admitted by preterm labor or preterm premature rupture of membranes. The vaginal microbial composition of women diagnosed as preterm premature rupture of membranes showed higher diversity than in that women diagnosed as preterm labor. In addition, the vaginal fluid of pregnant woman with a risk of preterm birth is dramatically altered in the proportions of Bacteroides, Prevotella and Weissella according to Lactobacillus dominance. In particular, in all the three groups classified according to the Lactobacillus dominance, among the top 20 major microbial groups, Bacteroides, Prevotella and Weissella showed the strong association with preterm birth.

It was associated with normal delivery as the dominance of Lactobacillus and Weissella was increased, and it was associated with premature delivery as the dominance of Bacteroides and Prevotella was increased. In particular, the depletion of Lactobacillus was related to the presence of pathogenic bacteria, prevalence of chorioamnionitis and low delivery week, and Weissella with high proportion in women with vaginal dysbiosis was related to delayed delivery. Moreover, the IL-7 concentration is associated with the low proportion of Lactobacillus and high proportion of Bacteroides. The present study further suggests that the reduction of Lactobacillus and the increase of Bacteroides which are associated with IL-7 that is an indicator related to preterm birth and the reduction of Weissella which is associated with IL-1β may be used as markers of preterm birth.

## Claims

1. A method of providing information for diagnosing a risk of preterm birth, comprising detecting a Weissella microorganism in a sample of pregnant woman.

2. The method of claim 1, further comprising detecting a microorganism selected from the group consisting of Lactobacillus, Bacteroides and Prevotella.

3. The method of claim 1, comprising
(a) extracting genomic DNA from a sample of pregnant woman,
(b) reacting the extracted genomic DNA with a primer specific for the Weissella microorganism, and
(c) amplifying the reactants.

4. The method of claim 3, wherein step (c) is performed through a polymerase reaction.

5. The method of claim 3, further comprising comparing the amount of amplified product of the step (c) with the amplified product of a sample of normal pregnant woman.

6. The method of claim 1, wherein the sample is a vaginal sample.

## Patentansprüche

1. Verfahren zum Bereitstellen von Informationen zur Diagnose eines Frühgeburtsrisikos, umfassend das Feststellen eines Weissella-Mikroorganismus in einer Probe einer schwangeren Frau.

2. Verfahren nach Anspruch 1, ferner umfassend das Feststellen eines Mikroorganismus, ausgewählt aus der Gruppe bestehend aus Lactobacillus, Bacteroides und Prevotella.

3. Verfahren nach Anspruch 1, umfassend
(a) Extrahieren genomischer DNA aus einer Probe einer schwangeren Frau,
(b) Umsetzen der extrahierten genomischen DNA mit einem für den Weissella-Mikroorganismus spezifischen Primer, und
(c) Amplifizieren der Reaktanten.

4. Verfahren nach Anspruch 3, wobei der Schritt (c) durch eine Polymerase-Reaktion ausgeführt wird.

5. Verfahren nach Anspruch 3, ferner umfassend das Vergleichen der Menge des amplifizierten Produkts aus Schritt (c) mit dem amplifizierten Produkt einer Probe einer normalen schwangeren Frau.

6. Verfahren nach Anspruch 1, wobei die Probe eine Vaginalprobe ist.

## Revendications

1. Procédé de fourniture d'informations pour le diagnostic d'un risque d'accouchement prématuré, comprenant la détection d'un micro-organisme Weissella dans un échantillon de femme enceinte.

2. Procédé selon la revendication 1, comprenant en outre la détection d'un micro-organisme choisi dans le groupe constitué de Lactobacillus, de Bacteroides et de Prevotella.

3. Procédé selon la revendication 1, comprenant
(a) l'extraction d'ADN génomique d'un échantillon de femme enceinte,
(b) la mise en réaction de l'ADN génomique extrait avec une amorce spécifique pour le micro-organisme Weissella, et
(c) l'amplification des réactifs.

4. Procédé selon la revendication 3, dans lequel l'étape (c) est réalisée par une réaction de polymérase.

5. Procédé selon la revendication 3, comprenant en outre la comparaison de la quantité de produit amplifié de l'étape (c) avec le produit amplifié d'un échantillon de femme enceinte normale.

6. Procédé selon la revendication 1, dans lequel l'échantillon est un échantillon vaginal.
